# EUROPEAN PATENT APPLICATION

(11) **EP 1 716 825 A1**
(43) Date of publication of application: **02.11.2006**
(21) Application number: 06008921.6
(22) Date of filing: 28.04.2006
(51) Int. Cl.: A61F 2/16

(54) **Intraocular lens having hydratable elastic part**

(30) Priority: 28.04.2005 JP 2005131524
(71) Applicant: Nidek Co., Ltd., Gamagori-shi, Aichi-ken 443-0035 (JP)
(72) Inventor: Sunada, Tsutomu, Toyohashi-shi Aichi-ken, 441-3102 (JP); Miura, Kyoko, Gamagori-shi Aichi-ken, 443-0037 (JP); Nakahata, Yoshihiro, Gamagori-shi Aichi-ken, 443-0011 (JP)
(74) Representative: Bauch-Koepe, Katharina Anna

(57) **Abstract**

An intraocular lens (1) capable of obtaining favorab7.e accommodative power, which is to be arranged inside a lens capsule as a replacement for a crystalline lens, includes a front-side and rear-side optical parts (2), (3) having predetermined refractive power and an elastic part (4) having predetermined elasticity by containing water to swell, wherein the elastic part is bonded to the optical parts so that optical axes of the optical parts coincide and so that the elastic part does not cover their optical zones, an elastic part thickness before containing water is determined so that an intraocular lens thickness after containing water completely to swell corresponds to a crystalline lens thickness at near vision, and a distance between the rear surface of the front-side optical part and the front surface of the rear-side-optical part of the intraocular lens arranged inside the lens capsule is changed according to relaxation and tension of a ciliary muscle.

## Description

### BACKGROUND OF THE INVENTION

### 1. Field of the Invention

The present invention relates to an intraocular lens used as a replacement for a crystalline lens.

### 2. Description of Related Art

Conventionally, there is known an intraocular lens which is arranged inside a lens capsule from which substances (a lens nucleus and a lens cortex) of an opaque crystalline lens have been removed for cataract treatment. Such an intraocular lens includes an optical part having predetermined refractive power and a support part for supporting the optical part inside the lens capsule. In addition, an art has been proposed recently for obtaining a replacement for the crystalline lens, which has accommodative power, by filling directly the lens capsule from which the opaque crystalline-lens substances have been removed with an injectable composition containing a hydrophilic polymer (refer to USP 6,413,262 corresponding to Japanese Patent Application Unexamined Publication No. 2001-252300).

However, in a method of filling the lens capsule with the injectable composition containing the hydrophilic polymer, there is a concern that the injectable composition could leak out of the lens capsule afterward. In addition, since refractive index of the hydrophilic polymer is smaller than that of a human crystalline lens, favorable visual acuity cannot be obtained even if the accommodative power is obtained by filling the lens capsule with the hydrophilic polymer.

### SUMMARY OF THE INVENTION

An object of the invention is to overcome the above problems and to provide an intraocular lens capable of obtaining favorable accommodative power.

To achieve the objects and in accordance with the purpose of the present invention, an intraocular lens to be arranged inside a lens capsule as a replacement for a crystalline lens includes a front-side optical part and a rear-side optical part having predetermined refractive power and an elastic part having predetermined elasticity by containing water to swell, wherein the elastic part is bonded to a rear surface of the front-side optical part and a front surface of the rear-side optical part so that respective optical axes of the front-side and rear-side optical parts coincide, and so that the elastic part does not cover optical zones of the front-side and rear-side optical parts, the elastic part is arranged so that a thickness of the elastic part before containing water is determined so that a thickness of the intraocular lens including the elastic part after containing water completely to swell corresponds approximately to a thickness of the crystalline lens at near vision, and the intraocular lens arranged inside the lens capsule is arranged so that a distance between the rear surface of the front-side optical part and the front surface of the rear-side optical part is changed according to relaxation and tension of a ciliary muscle.

Additional objects and advantages of the invention are set forth in the description which follows, are obvious from the description, or may be learned by practicing the invention. The objects and advantages of the invention may be realized and attained by the intraocular lens in the claims.

### BRIEF DESCRIPTION OF THE DRAWINGS

The accompanying drawings, which are incorporated in and constitute a part of this specification, illustrate embodiments of the present invention and, together with the description, serve to explain the objects, advantages and principles of the invention. In the drawings,
Figs. 1A and 1B are views showing a schematic configuration of an intraocular lens consistent with the preferred embodiment of the present invention;
Fig. 2 is a view showing the present intraocular lens being separated into respective components;
Figs. 3A and 3B are views showing the present intraocular lens being arranged in a lens capsule; and
Figs. 4A to 4C are views showing modified embodiments of the present invention.

### DETAILED DESCRIPTION OF THE PREFERRED EMBODIMENTS

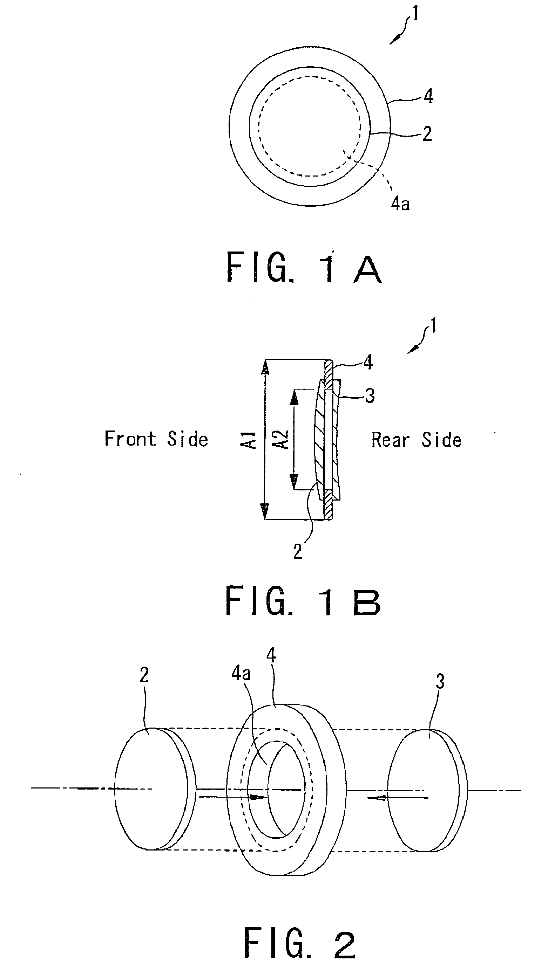

A detailed description of one preferred embodiment of an intraocular lens consistent with the present invention is provided below with reference to the accompanying drawings. Figs. 1A and 1B are views showing a schematic configuration of an intraocular lens 1 consistent with the preferred embodiment of the present invention, Fig. 1A being an external view of the intraocular lens 1 from the front and Fig. 1B being a sectional view thereof from the side. Fig. 2 is a view showing the intraocular lens 1 being separated into respective components. Besides, in the following description, the term "front" refers to a corneal side when arranged inside an eye (inside a lens capsule), and the term "rear" refers to a fundus side when arranged inside the eye.

The intraocular lens 1 includes a front-side optical part 2 and a rear-side optical part 3 having predetermined refractive power, and an elastic part 4 which connects the optical part 2 with the optical part 3. The elastic part 4 is hydrophilic, and when arranged inside the lens capsule, it gelates to be soft by containing water of body fluid such as aqueous humor to swell, so as to have predetermined elasticity. In addition, the elastic part 4 is formed into an approximate cylinder (an approximate ring) having an opening part 4a (an internal space) so that the elastic part 4 does not cover respective optical zones of the optical parts 2 and 3 through which a light bundle passes. Besides, a thickness of the elastic part 4 before containing water is determined so that a thickness of the intraocular lens 1 including the elastic part 4 after containing water completely to swell corresponds approximately to a thickness of a human crystalline lens (lens capsule) at near vision. In addition, an outer diameter A1 of the elastic part 4 before containing water is determined so that the outer diameter A1 of the elastic part 4 after containing water completely to swell corresponds approximately to an internal diameter of the human crystalline lens (lens capsule) at near vision. An internal diameter A2 of the elastic part 4 before containing water is determined so that the internal diameter A2 of the elastic part 4 after containing water completely to swell becomes larger (longer) than respective diameters of the optical zones of the optical parts 2 and 3 (i.e., respective effective optical diameters of the optical parts 2 and 3) and smaller (shorter) than respective diameters of the optical parts 2 and 3.

The elastic part 4 as mentioned above, which is made from a high hydrophilic material with hydrophilicity enough to dissolve in water as a main material and a hydrophilic material with hydrophilicity not enough to dissolve in water or a hydrophobic material as a secondary material, is obtained by blending respective monomers of the main material and the secondary material as appropriate to copolymerize. As the main material, employed is vinyl pyrrolidone, polyethyleneglycol or the like. As the secondary material, employed are (meth)acrylates such as hydroxyethyl methacrylate, ethyleneglycolmethyl ether methacrylate and phenylethyl methacrylate, acrylamides, vinylactams or the like. In the elastic part 4 to be prepared (in a base material thereof), the content of the main material is preferably not less than approximately 60 wt% and not more than approximately 95 wt%, and more preferably, not less than approximately 80 wt% and not more than approximately 90 wt%. In the elastic part 4 to be prepared (in the base material), the content of the secondary material is preferably not less than approximately 5 wt% and not more than approximately 40 wt%, and more preferably, not less than approximately 10 wt% and not more than approximately 20 wt%.

Additionally, when these main and secondary materials are copolymerized to obtain the elastic part 4, a cross-linking agent, a polymerization initiator or the like is employed as required. As the cross-linking agent, employed is dimethacrylate ester such as ethyleneglycol dimethacrylate, polyethyleneglycol dimethacrylate, diethyleneglycol dimethacrylate and triethyleneglycol dimethacrylate, or another material which is usable as a cross-linking agent in intraocular lens making. In addition, as the polymerization initiator, employed is a material which is usable as a polymerization initiator in intraocular lens making, such as azobisisobutyronitrile, azobisisobutyrovaleronitrile, benzoin, and methylorthobenzoyl benzoate. The cross-linking agent and the polymerization initiator are employed respectively in trace amounts, of which the content is not more than approximately 1 wt% with respect to the total weight.

Additionally, for the elastic part 4 made by employing these various materials, the water content is preferably approximately 70% to approximately 95%, and more preferably, approximately 80% to approximately 90%, the rate of swelling is preferably approximately 200% to approximately 400%, and the blending ratio of the various materials is determined so that the elastic part 4 has elasticity at the same level as the human crystalline lens when brought to a state of containing water completely to swell.

Meanwhile, for the optical parts 2 and 3, employed is a known material for intraocular lens including an unfoldable intraocular lens material such as polymethyl methacrylate and a foldable intraocular lens material such as an acrylic resin made from a copolymer of methacrylate ester and acrylic ester, silicone or the like.

In addition, curvature of the optical parts 2 and 3, respectively, is determined so that the intraocular lens 1 has predetermined refractive power as a whole. Besides, in the intraocular lens 1 consistent with the present embodiment, the optical part 2 is a convex lens and the optical part 3 is a concave lens; however, they are not limited thereto, and it is essential only that accommodation function of the intraocular lens 1 is carried out favorably by combining various lenses such as a convex lens, a concave lens and a meniscus lens as appropriate. In addition, since the optical parts 2 and 3 are bonded to the elastic part 4, it is preferable that at least respective bonding areas of a rear surface of the optical part 2 and of a front surface of the optical part 3 are flat.

For a bonding agent for bonding the optical parts 2 and 3 to the elastic part 4, it is essential only to have bonding force so as not to result in separation of the optical parts 2 and 3 from the elastic part 4, nor to result in axis deviation because of swelling of the elastic part 4 by containing water after the intraocular lens 1 is arranged inside the lens capsule. For such a bonding agent, employed is a known bonding agent which has high biocompatibility and does not affect the materials of the optical parts 2 and 3 and the elastic part 4. In addition, a bonding agent containing monomers which are copolymerizable with both of the optical parts 2 and 3 and the elastic part 4 may be employed.

Incidentally, the optical parts 2 and 3 and the elastic part 4 are bonded so that respective optical axes of the optical parts 2 and 3 coincide with the central axis of the elastic part 4 (opening part 4a). In addition, it is preferable for them to be bonded leaving a partial clearance such that the body fluid can infiltrate into the opening part 4a of the elastic part 4.

The optical parts 2 and 3 are respectively made by pouring a material solution thereof (a mixture solution of monomers) into a mold in a predetermined shape to be subjected to polymerization and curing. Alternatively, they are made by subjecting the material solution thereof to polymerization and curing to form into a plate, and then cutting the plate to have a predetermined shape. The elastic part 4 is made in the same manner.

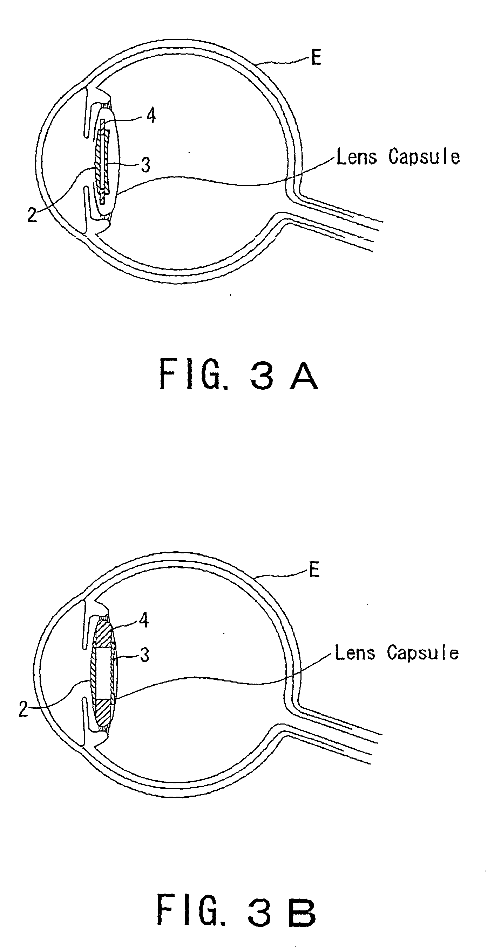

In a front surface of the lens capsule of a patient' s eye E, formed is an incision of a size such that through which the intraocular lens 1 before containing water can be inserted into the lens capsule, and substances of the crystalline lens is removed by phacoemulsification. Then, the intraocular lens 1 before containing water is inserted (injected) through the incision to be arranged inside the lens capsule (see Fig. 3A). The intraocular lens 1 before containing water is small (thin), so that respective incisions in a cornea and the lens capsule can be made smaller. Further, if the optical parts 2 and 3 are foldable, the respective incisions can be made much smaller.

The intraocular lens 1 (the elastic part 4) arranged inside the lens capsule contains water of the body fluid to swell, being less prone to coming out from the incision in the lens capsule.

Further, when the intraocular lens 1 (the elastic part 4) is brought to the state of containing water completely to swell after a lapse of predetermined time from the arrangement, the outer diameter of the intraocular lens 1 (the elastic part 4) comes to correspond approximately to the internal diameter of the lens capsule, allowing the intraocular lens 1 to be held in the lens capsule (see Fig. 3B). In addition, the opening part 4a of the elastic part 4 is filled with the body fluid. When the elastic part 4 is brought to the state of containing water completely to swell, it comes to have the elasticity at the same level as the human crystalline lens.

When the elastic part 4 contains water completely to swell inside the lens capsule, and the intraocular lens 1 corresponds approximately to the crystalline lens in form, e.g., the thickness of the intraocular lens 1 corresponds approximately to the thickness of the crystalline lens at near vision, a front surface of the optical part 2 comes into contact with a front side of the lens capsule and a rear surface of the optical part 3 comes into contact with a rear side of the lens capsule. Owing to the intraocular lens 1 brought to such a state, when the thickness of the lens capsule changes according to relaxation and tension of a ciliary muscle, a distance between the optical part 2 and the optical part 3 changes according to the thickness change, so that a focal length of the intraocular lens 1 changes. In other words, at the time of far vision, the ciliary muscle is relaxed and the lens capsule is pulled outward to become thinner, according to which, also the elastic part 4 stretches outward to become thinner and the distance between the rear surface of the optical part 2 and the front surface of the optical part 3 becomes smaller (shorter). As a result of this, an image of an object gazed at far vision is formed on a retina. Contrarily, at the time of near vision, the ciliary muscle gets tense and the lens capsule becomes thicker, according to which, the elastic part 4 regains the thickness at the time of swelling (the thickness at the maximum) and the distance between the rear surface of the optical part 2 and the front surface of the optical part 3 becomes greater (longer). As a result of this, an image of an object gazed at near vision is formed on the retina.

Incidentally, in the intraocular lens 1 consistent with the present embodiment, only the body fluid is interposed between the optical part 2 and the optical part 3 to increase a difference of refractive index, whereby a change in the distance between the optical part 2 and the optical part 3, which is necessary for accommodation, becomes acceptable even small. Accordingly, flexibility in optical design for the intraocular lens 1 increases and favorable accommodative power can be obtained.

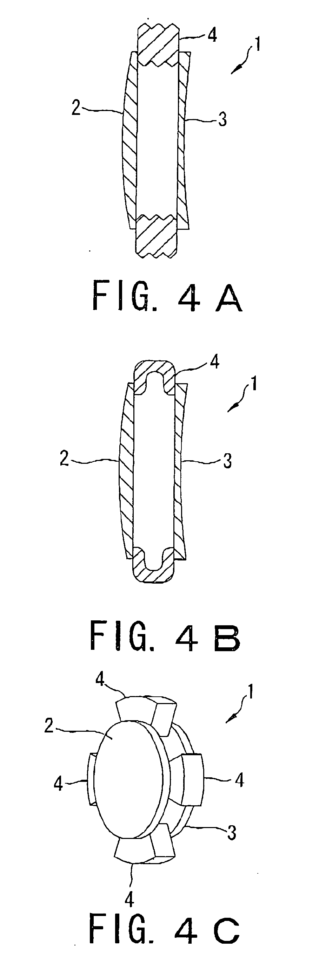

As described above, in the present embodiment, the elastic part 4 is made to form a simple hollow approximate cylinder (approximate ring); however, it is not limited thereto, and it may be made to have elasticity depending on its form in addition to the elasticity depending on its material. For example, as shown in Fig. 4A, the elastic part 4 in a state of containing water completely to swell may have an accordion shape in cross section, or as shown in Fig. 4B, the elastic part 4 in a state of containing water completely to swell may have an approximately arc shape in cross section.

Additionally, in order to make it easier for the body fluid to infiltrate into the opening part 4a, a hole, a notch or the like may be provided partially to the elastic part 4. Further, as shown in Fig. 4C, the elastic part 4 may be formed into a hollow approximate cylinder which is separated into a plurality of pieces. Such a form is assumed to be of hollow approximate cylinder in a broad sense.
The foregoing description of the preferred embodiments of the invention has been presented for purposes of illustration and description. It is not intended to be exhaustive or to limit the invention to the precise form disclosed, and modifications and variations are possible in the light of the above teachings or may be acquired from practice of the invention. The embodiments chosen and described in order to explain the principles of the invention and its practical application to enable one skilled in the art to utilize the invention in various embodiments and with various modifications as are suited to the particular use contemplated. It is intended that the scope of the invention be defined by the claims appended hereto.

## Claims

1. An intraocular lens (1) to be arranged inside a lens capsule as a replacement for a crystalline lens comprising:
a front-side optical part (2) and a rear-side optical part (3) having predetermined refractive power; and
an elastic part (4) having predetermined elasticity by containing water to swell,
wherein the elastic part is bonded to a rear surface of the front-side optical part and a front surface of the rear-side optical part so that respective optical axes of the front-side and rear-side optical parts coincide, and so that the elastic part does not cover optical zones of the front-side and rear-side optical parts,
the elastic part is arranged so that a thickness of the elastic part before containing water is determined so that a thickness of the intraocular lens including the elastic part after containing water completely to swell corresponds approximately to a thickness of the crystalline lens at near vision, and
the intraocular lens arranged inside the lens capsule is arranged so that a distance between the rear surface of the front-side optical part and the front surface of the rear-side optical part is changed according to relaxation and tension of a ciliary muscle.

2. The intraocular lens according to claim 1, wherein the elastic part is formed into a hollow approximate cylinder having the thickness in a direction of the respective optical axes of the front-side and rear-side optical parts.

3. The intraocular lens according to claim 1 or 2, wherein the elastic part is arranged so that an outer diameter of the elastic part before containing water is determined so that the outer diameter of the elastic part after containing water completely to swell corresponds approximately to an internal diameter of the lens capsule.

4. The intraocular lens according to any one of claims 1 to 3, wherein the elastic part is arranged so that an internal diameter of the elastic part before containing water is determined so that the internal diameter of the elastic part after containing water completely to swell becomes larger than diameters of the respective optical zones of the front-side and rear-side optical parts and smaller than diameters of the front-side and rear-side optical parts.

5. The intraocular lens according to any one of claims 1 to 4, wherein the elastic part is bonded to the front-side and rear-side optical parts by means of a bonding agent containing monomers which are copolymerizable with both of the front-side and rear-side optical parts and the elastic part.

6. The intraocular lens according to any one of claims 1 to 5, wherein the front-side optical part is a convex lens, and the rear-side optical part is a concave lens.

7. The intraocular lens according to any one of claims 1 to 6, wherein the front-side and rear-side optical parts are foldable.
